# EUROPÄISCHE PATENTSCHRIFT

(11) **EP 0 448 816 B1**
(45) Veröffentlichungstag und Bekanntmachung des Hinweises auf die Patenterteilung: **28.08.1996**
(21) Anmeldenummer: 90124696.7
(22) Anmeldetag: 19.12.1990
(51) Int. Cl.: G01N 1/00, G01N 1/22, G01N 27/62, G01N 33/18, G01N 1/28

(54) **Vorrichtung zur Bestimmung flüchtiger Stoffe in einer Flüssigkeit**
Apparatus for determining volatile substances in a liquid
Appareil pour déterminer des substances volatiles dans un liquide

(30) Priorität: 07.03.1990 DE 4007064
(43) Veröffentlichungstag der Anmeldung: 02.10.1991
(73) Patentinhaber: MANNESMANN Aktiengesellschaft, D-40213 Düsseldorf (DE)
(72) Erfinder: Kahl, Melchior, W-5060 Bergisch Gladbach (DE); Kitzelmann, Dieter, Dr., W-5300 Bonn 3 (DE)
(74) Vertreter: Meissner, Peter E., Dipl.-Ing.

(56) Entgegenhaltungen:
- DE-C- 2 932 436
- US-A- 3 942 792
- US-A- 4 330 385
- US-A- 4 546 640

## Beschreibung

Die Erfindung betrifft eine Vorrichtung zur Erfassung von flüchtigen Inhaltsstoffen (Gase bzw. Dämpfe) in Wasser. Hierbei wird das zu analysierende Wasser kontinuierlich durch einen Phasentauscher (Stripper) geleitet, wo die flüchtigen Bestandteile mit einem Trägergasstrom ausgetragen werden. Der mit der Substanz angereicherte Gasstrom wird dann einem Analysengerät zugeführt, welches diese Substanz nachweist.

Zur rechtzeitigen Erkennung plötzlich und unbeabsichtigt aufgetretener Emission von wassergefährdenden Substanzen im Kühlwasser oder in Produktionsabwässern sind Analysenverfahren, die zumindest einen qualitativen Frühnachweis erbringen können, von großer Bedeutung.

Ein typisches und daher häufig auftretendes Problem ist die rechtzeitige Detektion von organischen Stoffen, wie z.B. aromatische und aliphatische Kohlenwasserstoffe, Ether, Ester, Alkohole, Ketone, Halogenkohlenwasserstoffe u.a. Analysenverfahren, die die organische Fracht in Wasser messen können, sind bekannt. Automatische Geräte messen z.B. den TC-Wert (Total Carbon), indem eine kleine Abwasserprobe in einem Pyrolyseofen thermisch/katalytisch zu CO₂ verbrannt wird. Die CO₂-Konzentration wird anschließend in einem Infrarot-Analysator erfaßt (1).

Es ist ferner bekannt, die organische Fracht über den chemischen Sauerstoffbedarf (CSB-Wert) zu bestimmen. Auch hierfür stehen kontinuierliche Meßverfahren zur on line-Analyse zur Verfügung.

Nachteilig ist, daß diese Geräte eine recht aufwendige Analyseprozedur durchführen und daher technologisch aufwendig und wartungsintensiv sind. Hinzu kommt, daß die Ansprechzeiten im Bereich mehrerer Minuten liegen, so daß eine rasche Alarmgabe bei plötzlich auftretenden Leckagen nicht möglich ist.

Ziel dieser Erfindung ist eine Vereinfachung einer solchen Meßeinrichtung sowie eine Verbesserung des Ansprechverhaltens durch die Kombination eines sehr schnellen Analysengerätes mit einer wirkungsvollen Strippvorrichtung. In der Strippvorrichtung werden die flüchtigen organischen Substanzen von der wäßrigen Phase in die Gasphase überführt.

Es ist bekannt, die Überführung von organischen Bestandteilen in die Gasphase nach der Headspace-Methode oder durch Strippvorrichtungen vorzunehmen und den Gehalt an organischen Stoffen in dieser Gasphase zu bestimmen.

Bei der Headspace-Methode nützt man nach dem Henry'schen Gesetz das Gleichgewichtsverhältnis der Konzentrationen an organischen Stoffen in Wasser und dem darüber befindlichen Gasraum aus. Diese Methode wird häufig in Kombination mit einem Gaschromatographen verwendet, da nur geringe Mengen des zu analysierenden Gasraumes vorhanden sind und für diesen Analysator auch nur benötigt werden.

Bei den bekannten Strippeinrichtungen wird z.B. ein geschlossener Trägergaskreislauf benutzt, um die organischen Bestandteile im Wasser vollständig abzureichern und entweder den Gasstrom direkt einem Gaschromatographen zuzuführen (2), oder bei nur geringen Mengen die organischen Substanzen in einer Kühlfalle oder an einem Adsorber anzureichern und anschließend durch Verdampfen bzw. Desorbieren dieses aufkonzentrierte Gas im Gaschromatographen zu messen (3/4). Die eben genannten Methoden sind ausschließlich Labormethoden und ermöglichen nur eine diskontinuierliche Messung.

Eine weitere Labormethode zur Bestimmung von Kohlenwasserstoffen in Wasser benutzt "in-situ" elektrochemisch erzeugten Wasserstoff als Strippgas mit anschließend gaschromatographischer Bestimmung der Kohlenwasserstoffe im Dampfraum der Probe (5/6).

Zur kontinuierlichen Überwachung von Wasser auf leichtflüchtige Halogenkohlenwasserstoffe ist ein Strippverfahren bekannt, bei dem in einem Durchflußgeber mit Syphon durch ein Rohr mit Löchern Luft in das Wasser einperlt. Der Durchflußgeber befindet sich in dem zu untersuchenden Wasserstrom. Die Stripperluft perlt hierbei im Überschuß durch die Löcher in das Wasser und reichert sich dabei mit den organischen Stoffen an. Ein Teil des Gases wird einem Analysengerät zwecks Bestimmung der organischen Substanzen zugeführt.

Beim bisherigen Verfahren ist nachteilig, daß je nach der Durchflußgeschwindigkeit des Wassers und je nach dem Wasserstand in der Durchflußapparatur mehr oder weniger viel Strippgas vom Wasserstrom mitgerissen wird. Im ungünstigen Falle wird alles Gas mitgerissen, so daß kein Gas zur Analyse zur Verfügung steht. Nachteilig ist ebenfalls, daß bei Verstopfungen auf der Abflußseite Wasser über die Löcher in die Gasleitung und somit in den Gasanalysator gelangen kann. Letztendlich kann, in Abhängigkeit vom Wasserstand im Durchflußgeber, der Gasraum über dem Wasser sehr groß sein, so daß Gas vom Analysengerät gemessen wird, welches nicht mit der Gaszusammensetzung über der aktuellen Wasserprobe identisch ist. Dies bedeutet jedoch eine Verfälschung der Meßergebnisse.

Ziel der vorliegenden Erfindung ist eine Verbesserung der Sprippingprozedur in Verbindung mit einem Gasanalysengerät z.B. bei der Messung von organischen Inhaltsstoffen in Wasser hinsichtlich eines raschen Stoffaustausches mit einer direkten Zuführung des aktuellen Strippgases zum Analysator, wobei der Strippvorgang nicht durch die Füllhöhe der Durchflußarmatur oder durch eine hohe Strömungsgeschwindigkeit in der Apparatur beeinträchtigt wird.

Weiterhin ist aus der US 4546640 ein Detektorsystem bekannt, bei welchem Strip Gas in ein quasi Entnahmerohr unterhalb der Füllhöhe desselben eingeleitet wird. Das Stripgas wird ohne Rücksicht auf Strömung in die Flüssigkeit eingeleitet, so daß der Strip-Vorgang nicht optimal ist. Aus der US 3942792 ist eine gattungsgemäße Anordnung bekannt, bei welcher in einem Entnahmerohr eine gezielte Strömung erzeugt wird. Innerhalb dieser Strömung wird das Strip-Gas quasi strömungsgeführt eingeleitet. Auch hierbei ist der Stripvorgang nicht optimiert, weil die Flüssigkeit über eine Siphonähnliche Anordnung wieder zurückfließt und ein Teil des Stripgases bei dieser Prozedur ungenutzt von der strömenden Flüssigkeit mitgerissen werden kann.

Der Erfindung liegt daher die Aufgabe zugrunde, die Stripping-Prozedur zu verbessern.

Diese Aufgabe wird, ausgehend von einem Gasanalysengerät mit einem zu der Flüssigkeit führenden Entnahmerohr erfindungsgemäß dadurch gelöst, daß das Entnahmerohr mit einem in die Flüssigkeit hineinragenden Tauchrohr verbunden ist, das einen mit dem Gaszuleitungsrohr verbundenen porösen Verteilerkörper umschließt, der von der Flüssigkeit seitlich angeströmt wird. Das Trägergas gelangt dabei über den Verteilerkörper in Form von feinverteilten Gasblasen in das vom Tauchrohr eingeschlossene Flüssigkeitsvolumen und strömt nach dem Durchperlen vollständig nach oben ab. Ein Teilstrom wird kontinuierlich zum Analysengerät zur Bestimmung der Meßkomponenten gefördert. Durch das Tauchrohr wird also das vom Trägergas durchperlte Flüssigkeitsvolumen eingeschlossen, so daß in der Flüssigkeit ein definiertes Volumen für den Stoffaustausch (Strippvorgang) zur Verfügung steht.

Der Verteilerkörper besteht aus einer schräg zur Strömungsrichtung angeordneten porösen Platte.

Das Tauchrohr ist an seinem unteren Ende auf der der strömenden Flüssigkeit zugewandten Seite abgeschrägt und erstreckt sich mit seinem innerhalb des Durchflußrohres angeordneten Teil über mindestens zwei Drittel des Durchflußrohrdurchmessers. Dadurch wird erreicht, daß auch bei niedrigem Füllstand in dem Durchflußrohr ein laufender Austausch mit neu zufließender Flüssigkeit möglich ist und gleichzeitig in Folge der auftretenden Turbulenzen im Tauchrohr eine Verschmutzung durch Feststoffteile im Wasser verhindert werden kann.

Vorteilhafterweise beträgt der Anstellwinkel der porösen Verteilerplatte 30°-85°, vorzugsweise 50°-70°.

Ferner hat es sich als günstig erwiesen, wenn der Abstand zwischen der schrägstehenden Verteilerplatte und der schrägen Unterseite des Tauchrohres so bemessen ist, daß das Flüssigkeitsvolumen im Tauchrohr gering ist und somit ein schneller Austausch erfolgt. Gleichzeitig wird dadurch gewährleistet, daß das Trägergas relativ schnell innerhalb des Entnahmerohres hochsteigen kann. Die Höhe des Flüssigkeitsniveaus im Tauchrohr wird im wesentlichen durch das Niveau im Durchflußrohr bestimmt, wenn kein Staudruck auf der abströmenden Seite überwunden werden muß. Im Hinblick auf die Minimierung des Wasservolumens, das vom Trägergas durchperlt werden muß, beträgt der Abstand a zwischen dem unteren Ende des Verteilerkörpers und dem unteren Ende des Entnahmerohres vorteilhaft 0,5 cm bis 1,5 cm.

Um bei Verstopfungen auf der Abflußseite oder bei hohem Staudruck im Rohrleitungssystem ein Eindringen der Flüssigkeit in die Gaszuführungsleitung zum Analysengerät zu vermeiden, ist das Tauchrohr zweckmäßig mit einem Überlauf versehen.

Besonders bewährt hat sich eine Ausführungsform, die aus einer Kombination der beschriebenen Strippvorrichtung mit einem Flammenionisationsdetektor (FID) besteht, an dessen gasseitigem Eingang Unterdruck aufrechterhalten wird, so daß das Stripp-Gas vom FID angesaugt wird.

Mit der Erfindung werden folgende Vorteile erzielt:
Die beschriebene Vorrichtung erlaubt eine kontinuierliche Überwachung von verdampfbaren anorganischen und organischen Substanzen in Wasser, z.B. von Lösungsmitteln. In Kombination mit einem Flammenionisationsdetektor (FID) als Gesamtkohlenstoffanalysator liegt die Ansprechzeit unterhalb von 10 s. Die Nachweisgrenze ist stoffabhängig und richtet sich nach der Flüchtigkeit und Löslichkeit in Wasser. Besonders empfindlich lassen sich die hydrophoben Kohlenwasserstoffe und die ökologisch Bedenklichen, chlorierten Kohlenwasserstoffe detektieren.

Die Nachweisgrenze für diese Stoffgruppen liegt unterhalb 10 µl/l. Damit ist die Kombination Stripper/FID auch bezüglich Nachweisempfindlichkeit einem TOC-Gerät überlegen. Schwerverdampfbare sowie salzartige Verbindungen können nicht erfaßt werden.

Im folgenden soll ein Ausführungsbeispiel der Erfindung anhand einer Zeichnung näher beschrieben werden:
Die zu untersuchende Flüssigkeit z.B. Wasser strömt hier von links nach rechts durch ein Durchflußrohr 1, das einen Siphon 2 bildet. Am Siphon 2 ist senkrecht zum Durchflußrohr 1 ein Tauchrohr 3 eingebaut. Das untere Ende des Tauchrohres 3 ist derart abgeschrägt, daß die Eintrittsfläche des Tauchrohres der Strömung zugewandt ist. Die Länge des schräg abgeflachten Tauchrohres ist so bemessen, daß auch bei niedrigem Füllstand im Durchflußrohr 1 ein schneller Austausch mit neu zufließendem Wasser möglich ist und gleichzeitig in Folge der auftretenden Turbulenzen im Tauchrohr eine Verschmutzung durch Feststoffanteile im Wasser verhindert wird. Praktisch wird dies dadurch erreicht, daß der in die Flüssigkeit hineinragende Teil sich über mehr als zwei Drittel des Durchmessers des Durchflußrohres 1 erstreckt. Am Tauchrohrkopf 5 zweigt ein Überlaufrohr 6 ab, welches im Falle eines Eindringens von Wasser über die Öffnung 7 dafür sorgt, daß das Wasser abfließt, bevor es das zum Gasanalysator 8 führende Entnahmerohr 9 erreichen kann. Das Gasanalysengerät ist hier ein Flammenionisationsdetektor (FID). Der FID wird an seinem Eingang mit Unterdruck betrieben, so daß die Meßgaskomponente durch das Entnahmerohr 9 angesaugt wird. Ein FID dieser Bauart wird in DE-PS 29 32 436 beschrieben.

Um zu vermeiden, daß bei sehr hohem Staudruck im Wassersystem zuviel Wasser hochsteigen kann, ist eine als Wassersperre dienende Blende 10 mit einer Öffnung von 2 bis 3 mm Durchmesser (Öffnung 7) vorgesehen.

Innerhalb des Tauchrohres 3 ist ein Gaszuleitungsrohr 11 mit einer schräggestellten porösen Verteilerplatte 12 angeordnet. Die poröse Verteilerplatte kann aus einer Glasfritte oder einer Sinterplatte oder aus einem Holzkörper bestehen. Alternativ kann ein feines Lochsieb verwendet werden. Der Anstellwinkel α der Verteilerplatte 12 (Winkel gegen die Horizontale) soll mindestens 30° betragen. Besonders bewährt hat sich ein Bereich zwischen 50° und 70°. Die Austrittsfläche des Verteilerkörpers ist der Strömung zugewandt; d.h. die Normale auf der Verteilerfläche hat eine der Strömung entgegengesetzte Komponente. Die Schrägstellung der Verteilerplatte 12 hat sich als besonders günstig erwiesen, da auf diese Weise feinverteilte nicht-koaleszierende Gasbläschen gebildet werden, die ein intensives Ausstrippen der Flüssigkeitsstrom (Wasser) enthaltenden flüchtigen Komponenten zur Folge haben.

Die Verteilerplatte 12 befindet sich knapp oberhalb des abgeschrägten Tauchrohrendes 4 und unterhalb des Flüssigkeitsspiegels im Tauchrohr 3, welches im Normalbetrieb durch den Flüssigkeitsstand im Durchflußrohrsiphon 2 vorgegeben ist. So beträgt der Abstand a zwischen dem unteren Ende der Verteilerplatte 12 und dem unteren Ende des Tauchrohres 3 0,5 bis 1,5 cm. Somit steht ein räumlich begrenztes, rasch austauschbares Wasservolumen für den Stripp-Prozeß zur Verfügung. Dies ist von wesentlicher Bedeutung für eine schnelle Ansprechzeit des FID 8, wenn sich die Konzentration der Meßgaskomponente ändert.

Als Trägergas wird normalerweise kohlenwasserstoff-freie atmosphärische Luft verwendet. Ebenfalls denkbar ist die Verwendung anderer Gase wie Stickstoff, Kohlendioxid oder Edelgase. Das Trägergas 13 gelangt über das Zuleitungsrohr 11 zu der Verteilerplatte 12 und steigt dort in Form von feinen Gasbläschen, angereichert mit den flüchtigen Inhaltsstoffen des Abwasserstromes, auf. Anschließend strömt die Gasmischung durch die Öffnung 7 der Wassersperre 10 in den Tauchrohrkopf 5. Ein Teil der Gasmischung wird durch das Entnahmerohr 9 vom FID 8 angesaugt, während der überschüssige Anteil durch das Überlaufrohr 6 ins Freie strömt.

Mit der beschriebenen Apparatur können Abwasserströme zwischen 30 und 300 l pro Stunde on-line überwacht werden. Dabei wird mit Trägergasströmen zwischen 30 und 100 l pro Stunde gearbeitet.

### Meßbeispiel

Abwassermengenstrom 150 l pro Stunde
Trägergasstrom (Luft) 100 l pro Stunde

Die zu untersuchende Meßkomponente bestand aus Chlorbenzol und Dichlormethandämpfen. Bei einer Stoffkonzentration von 50 µl/l Dichlormethan wurde im FID eine organische Kohlenstoffmenge von 320 mg/m³ gemessen. Die Nachweisgrenze betrug 0,4 µl/l.

Bei der Messung von Chlorbenzol betrug die Stoffkonzentration im Abwasserstrom 5 µl/l. Im FID wurde eine organische Kohlenstoffmenge von 210 mg/m³ gemessen. Die Nachweisgrenze betrug hier 0,3 µl/l. Die Ansprechzeit der Meßvorrichtung betrug bei einem Innendurchmesser von 4 mm des Entnahmerohrs 9 0,75 s/m Rohrlänge. Die Ansprechzeit des FID 8 wächst entsprechend mit zunehmender Länge des Entnahmerohrs 9. Im praktischen Betrieb kann eine Ansprechzeit von 3 bis 10 s erreicht werden. Sämtliche Messungen wurden bei Zimmertemperatur durchgeführt.
(1) G. Axt: Ein Gerät zur kontinuierlichen Messung des org. Kohlenstoffs im Trink- und Abwasserbereich: vom Wasser 36 (1969), 328-339.
(2) Wang, T.; et.al: "Determination of volatile halocarbons in water by purge-closed loop gas cromatography", Bull. Envision. Contam. Toxicol., 32 (4), 429-438.
(3) Okuno, T.; et.al: "Study on measurements of low boiling point chlorinated hydrocarbons in sea water", Hyogo-ken Kogai Kenkijusho Kenkyu Hokoku, 10, 8-14.
(4) Dowty, B.; et.al: "Automated gaschromatographic procedure to analyze volatile organics in water and biological fluids", Anal. Chem., 48 (6), 946-949.
(5) US-Patent 3 927 978, 23. Dez. 1975.
(6) US-Patent Appl. US 525 430, 20. Nov. 1974.

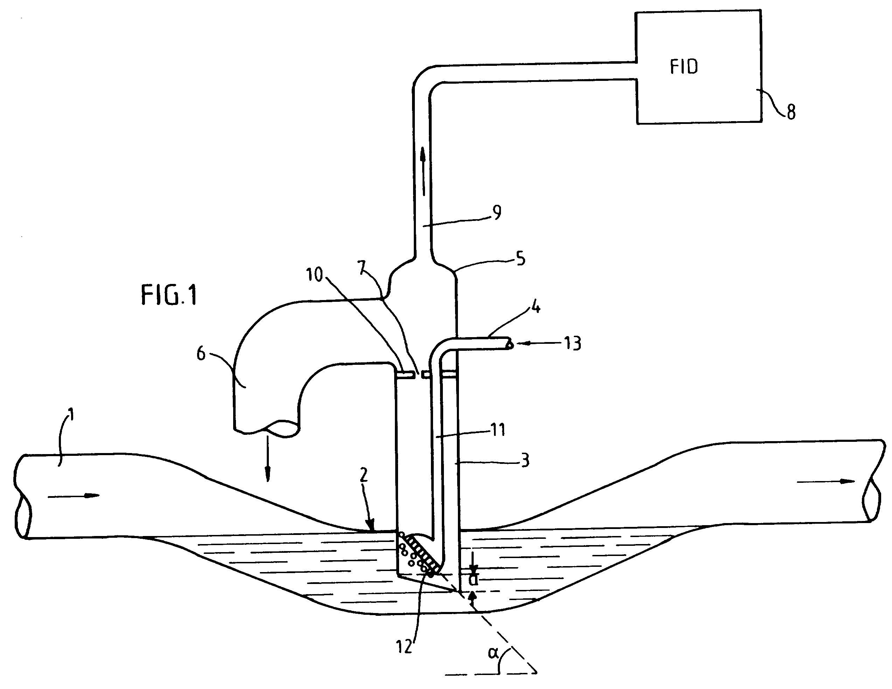

## Patentansprüche

1. Vorrichtung zur Bestimmung flüchtiger Gas- bzw. Dampfkomponenten in einer strömenden Flüssigkeit, bestehend aus einem Gasanalysegerät (8), das über ein Entnahmerohr (9) mit der Flüssigkeit in Verbindung steht, wobei durch ein Gaszuleitungsrohr (11) ein Trägergas (13) in die strömende Flüssigkeit einperit und dabei die zu messenden Gaskomponenten freisetzt, die zusammen mit dem Trägergas (13) durch das Entnahmerohr (9) dem Gasanalysegerät (8) zugeführt werden, wobei das Entnahmerohr (9) mit einem in die strömende Flüssigkeit hineinragenden Tauchrohr (3) verbunden ist, welches bezüglich seiner Austrittsfläche der Strömung zugewandt ist, und das Tauchrohr einen mit dem Gaszuleitungsrohr (11) verbundenen porösen Verteilerkörper (12) für die Zuführung des Trägergases (13) in Form fein verteilter Gasblasen derart umschließt, daß die Austrittsfläche des Verteilerkörpers ebenfalls der Strömung zugewandt ist,
dadurch gekennzeichnet,
daß der Verteilerkörper (12) aus einer schräg zur Strömungsrichtung angeordneten porösen Platte besteht, und daß die strömende, zu analysierende Flüssigkeit in einem Durchflußrohr (1) geführt wird, daß das Tauchrohr (3) an seinem unteren Ende (4) auf der der strömenden Flüssigkeit zugewandten Seite abgeschrägt ist und sich mit seinem innerhalb des Durchflußrohres (1) angeordneten Teil über mindestens 2/3 des Durchflußrohrdurchmessers erstreckt.

2. Vorrichtung nach Anspruch 1,
dadurch gekennzeichnet,
daß der Einstellwinkel α der porösen Verteilerplatte (12) 30° bis 85°, vorzugsweise 50° bis 70° beträgt.

3. Vorrichtung nach Anspruch 2,
dadurch gekennzeichnet,
daß der Abstand a zwischen dem unterem Ende des Verteilerkörpers (12) und dem unterem Ende (4) des Tauchrohres 0,5 cm bis 1,5 cm beträgt.

4. Vorrichtung nach Anspruch 3,
dadurch gekennzeichnet,
daß das Tauchrohr (3) einen Überlauf (6) aufweist.

5. Vorrichtung nach Anspruch 4,
dadurch gekennzeichnet,
daß das Gasanalysengerät (1) ein Flammenionisationsdetektor ist, an dessen gasseitigem Eingang Unterdruck herrscht.

## Claims

1. An apparatus for determining volatile gas or vapour constituents in a flowing liquid, consisting of a gas analyser (8) which communicates with the liquid via a sampling tube (9), wherein a carrier gas (13) bubbles into the flowing liquid through a gas inlet pipe (11) and in so doing releases the gas constituents to be measured, which are supplied to the gas analyser (8) together with the carrier gas (13) through the sampling tube (9), the sampling tube (9) being connected to a dip tube (3) which protrudes into the flowing liquid, the exit surface of which dip tube faces the flow, and the dip tube surrounds a porous distributor element (12), connected to the gas inlet pipe (11), for supplying the carrier gas (13) in the form of finely distributed gas bubbles such that the exit surface of the distributor element likewise faces the flow,
characterised in that the distributor element (12) consists of a porous plate arranged obliquely to the direction of flow, and that the flowing liquid which is to be analysed is guided in a throughflow tube (1), that the dip tube (3) is sloped at its lower end (4) on the side facing the flowing liquid and its part located within the throughflow tube (1) extends over at least 2/3 of the diameter of the throughflow tube.

2. An apparatus according to Claim 1, characterised in that the setting angle a of the porous distributor plate (12) is 30° to 85°, preferably 50° to 70°.

3. An apparatus according to Claim 2, characterised in that the distance a between the lower end of the distributor element (12) and the lower end (4) of the dip tube is 0.5 cm to 1.5 cm.

4. An apparatus according to Claim 3, characterised in that the dip tube (3) has an overflow (6).

5. An apparatus according to Claim 4, characterised in that the gas analyser (1) is a flame ionisation detector, to the gas-side inlet of which a partial vacuum is applied.

## Revendications

1. Dispositif pour déterminer des composants de gaz ou de vapeur volatils dans un liquide qui s'écoule, constitué d'un appareil d'analyse de gaz (8) qui est en liaison avec le liquide par l'intermédiaire d'un tube de prélèvement (9), un gaz porteur (13) étant introduit sous forme de bulles dans le liquide qui s'écoule, à travers un tube d'amenée de gaz (11) et libérant les composants de gaz à mesurer, qui sont amenés conjointement avec le gaz porteur (13) à travers le tube de prélèvement (9) dans l'appareil d'analyse de gaz (8), le tube de prélèvement (9) étant relié à un tube plongeur (3) faisant saillie dans le liquide qui s'écoule, tube plongeur qui est en regard de l'écoulement relativement à sa face de sortie, et qui entoure un corps distributeur (12) poreux relié au tube d'amenée de gaz (11), pour l'amenée du gaz porteur (13) sous forme de bulles de gaz finement reparties, de sorte que la face de sortie du corps distributeur est également en regard de l'écoulement,
caractérisé en ce que le corps distributeur (12) est constitué d'une plaque poreuse agencée de façon inclinée par rapport à la direction d'écoulement, et en ce que le liquide à analyser, qui s'écoule, est guidé dans un tube d'écoulement (1), en ce que le tube plongeur (3) est biseauté, à son extrémité inférieure (4), sur la face en regard du liquide qui s'écoule et s'étend, par sa partie agencée à l'intérieur du tube d'écoulement (1), sur au moins 2/3 du diamètre du tube d'écoulement.

2. Dispositif selon la revendication 1,
caractérisé en ce que l'angle de positionnement a de la plaque distributrice poreuse (12) est situé entre 30° et 85°, de préférence entre 50° et 70°.

3. Dispositif selon la revendication 2,
caractérisé en ce que l'écartement a entre l'extrémité inférieure du corps distributeur (12) et l'extrémité inférieure (4) du tube plongeur est situé entre 0,5 cm et 1,5 cm.

4. Dispositif selon la revendication 3,
caractérisé en ce que le tube plongeur (3) présente un trop-plein (6).

5. Dispositif selon la revendication 4,
caractérisé en ce que l'appareil d'analyse de gaz (1) est un détecteur d'ionisation de flammes, à la face d'entrée de gaz duquel règne une dépression.
